# EUROPEAN PATENT APPLICATION

(11) **EP 1 997 477 A1**
(43) Date of publication of application: **03.12.2008**
(21) Application number: 08009942.7
(22) Date of filing: 30.05.2008
(51) Int. Cl.: A61K 9/00, A61K 9/51, A61K 47/42

(54) **Anti-acne skin agent for external use**

(30) Priority: 31.05.2007 JP 2007144542
(71) Applicant: FUJIFILM Corporation, Minato-ku Tokyo (JP)
(72) Inventor: Aimi, Makiko, Ashigarakami-gun Kanagawa 258-8538 (JP); Ogiwara, Kazutaka, Ashigarakami-gun Kanagawa 258-8538 (JP)
(74) Representative: HOFFMANN EITLE

(57) **Abstract**

It is an object of the present invention to provide an anti-acne skin agent for external use which comprises highly safe protein nanoparticles having high transparency due to the small particle size, moisturizing properties, and high permeability into skin. The present invention provides an anti-acne skin agent for external use, which comprises protein nanoparticles containing an active ingredient.

## Description

### Technical Field

The present invention relates to an anti-acne skin agent for external use, which comprises protein nanoparticles containing an active ingredient.

### Background Art

Generally, acne comes out due to the clogging of the secreted sebum in the hair follicle. Epidemiologically, acne is a disease that over 90% of Japanese people experience. Acne becomes worst during adolescence. However, it is not rare that acne newly comes out even in the twenties. It is said that lack of sleep, stress, diet, and the like act as complicating factors for acne. In addition, oil contained in cosmetics and the like is also considered to be a complicating factor for acne.

However, makeup has a great influence on the state of patients' mind. Thus, if acne-friendly makeup is performed on patients suffering from acne while the treatments of acne are performed on the patients, the QOL of the patients can quickly be improved, as well as the improvement of acne.

Methods for treating acne include: administrations of internal medicines such as vitamins or antibiotics; external treatments using external preparations formed by mixing a sebum-suppressing and/or absorbing component, a keratin-softening and/or peeling component, an antibacterial and/or bactericidal component such as sulfur, an anti-inflammatory component, a vitamin A derivative such as retinoic acid, and the like; and other treatments such as chemical peeling. These treatments have demonstrated certain effects. However, they have not yet demonstrated sufficient effects, or have been problematic in terms of degeneration of the aforementioned components in formulations. Thus, a further improvement has been desired.

Treatments such as chemical peeling or the use of retinoic acid have been problematic in that they promote the dryness of the skin. Hence, the combined use of cosmetics having moisturizing effects with the aforementioned treatments is recommended. It is said that moisturizing agents containing no oils, such as those containing hyaluronic acid or amino acid as a main component, are preferable. However, these agents have not had sufficient moisturizing effects.

In addition, in view of the dosage forms of skin agents for external use comprising the above agents, lotions are likely to drip when applied to the skin. Also, in the cases of gels, agents are unlikely to be released from bases so that medicinal effects are not sufficiently exhibited in some cases.

Regarding cosmetics, more obvious skin-improving effects have been required in recent years. Manufactures have been attempting to improve the functionality and usability of their own products and to differentiate their own products from competitive products by applying a variety of technologies such as nanotechnology. In general, the stratum corneum serves as a barrier for the skin. Thus, medicines are unlikely to permeate therethrough into the skin. In order to obtain sufficient skin-improving effects, it is essential to improve the skin permeability of active ingredients. Moreover, it is difficult to formulate many active ingredients due to poor preservation stability or tendency to result in skin irritancy, although they are highly effective to the skin. In order to solve the above problems, a variety of fine particle materials have been under development for the improvement of transdermal absorption and preservation stability, reduction of skin irritancy, and the like. Recently, a variety of fine particle materials such as ultrafine emulsions and liposomes have been studied (e.g., Mitsuhiro Nishida, Fragrance Journal, November, 17 (2005)).

With the use of polymeric materials instead of emulsified products or liposomes, it can be expected that remarkable improvement in preservation stability and in *in vivo* particle stability will be achieved due to the structure of such material. However, in most studies, synthetic polymers obtained by, for example, emulsion polymerization are used, so that it is required to obtain safer carriers. For instance, in JP Patent Publication (Kokai) 2002-308728 A, an anti-acne skin agent for external use in nanoparticle form, which is obtained with the use of a polymer material, is proposed. In JP Patent Publication (Kokai) 2004-244420 A, crosslinked polymer nanoparticles comprising a skin care component are proposed. Since these are emulsified products obtained with the use of surfactants or polymeric substances comprising monomers or macromers (synthetic polymers having polymeric groups), these are suspected to be unsafe.

In Hiroyuki Tsujimoto, Drug Delivery System, 21-4, 405 (2006), lactic acid-glycolic acid copolymer (PLGA) nanoparticles, such polymer being a biocompatible polymer, are proposed. However, PLGA is likely to be hydrolyzed, which is problematic in terms of preservation stability. In addition, when PLGA is hydrolyzed *in vivo,* lactic acid is produced, resulting in negative influences.

Natural polymers exhibit high structural stability comparable to that of synthetic polymers. In addition, natural polymers are much safer than synthetic polymers and thus they are advantageous as DDS carriers. However, compared with synthetic polymers, there are problems in methods for producing particles of natural polymer carriers. Spray drying, lyophilization, and jet milling can be applied to natural polymer particle production methods. However, in most cases, natural polymer particles have micron-level particle sizes. Thus, it is difficult to control such particle sizes.

### Disclosure of the Invention

It is an object of the present invention to solve the above problems of the prior art techniques. Specifically, it is an object of the present invention to provide an anti-acne skin agent for external use which comprises highly safe protein nanoparticles having high transparency due to the small particle size, moisturizing properties, and high permeability into skin.

As a result of intensive studies directed towards achieving the aforementioned object, the present inventors prepared protein nanoparticles containing an active ingredient and applied the prepared protein nanoparticles to the skin. As a result, they demonstrated that the protein nanoparticles are highly safe and have high transparency, moisturizing properties, and favorable permeability into skin. The present invention has been completed based on the above findings.

That is to say, the present invention provides an anti-acne skin agent for external use, which comprises protein nanoparticles containing an active ingredient.

Preferably, the average particle size of the protein nanoparticles is 10 to 100 nm.

Preferably, the anti-acne skin agent for external use of the present invention contains protein nanoparticles in an amount of 0.01% to 50% by weight.

Preferably, protein nanoparticles contain an active ingredient in a weight that is 0.1 % to 100% of the protein weight.

Preferably, the active ingredient is at least one selected from the group consisting of antioxidant components, moisturizing components, reactive oxygen-removing agents, anti-inflammatory agents, antibacterial and/or bactericidal agents, sebum-suppressing and/or absorbing agents, anti-aging agents, collagen synthesis promoters, anti-wrinkle agents, vitamins, minerals, and amino acids.

Preferably, the active ingredient is an ionic substance or a fat-soluble substance.

Preferably, the protein is at least one selected from the group consisting of collagen, gelatin, acid-treated gelatin, albumin, ovalbumin, casein, transferrin, globulin, fibroin, fibrin, laminin, fibronectin, and vitronectin.

Preferably, the protein is subjected to crosslinking treatment during and/or after nanoparticle formation.

Preferably, crosslinking treatment is carried out by an enzyme.

The enzyme is not particularly limited so long as it has a function of crosslinking a protein, and preferably transglutaminase is used.

Preferably, the anti-acne skin agent for external use of the present invention comprises casein nanoparticles prepared by the following steps (a) to (c):
(a) mixing casein with a basic aqueous medium at pH of from 8 to less than 11;
(b) adding at least one active ingredient to the solution obtained in step (a); and
(c) injecting the solution obtained in step (b) into an aqueous medium at pH of 3.5 to 7.5:

Preferably, the anti-acne skin agent for external use of the present invention comprises casein nanoparticles prepared by the following steps (a) to (c):
(a) mixing casein with a basic aqueous medium at pH of from 8 to less than 11;
(b) adding at least one active ingredient to the solution obtained in step (a); and
(c) lowering the pH of the solution obtained in step (b) to pH value which is different from the isoelectric point by 1 or more units.

Particles containing an active ingredient in the anti-acne skin agent for external use of the present invention are nanoparticles, and thus they are highly absorbable. In addition, according to the present invention, since protein nanoparticles are used, there is no need to use chemical crosslinking agents or synthetic surfactants for production of the agent. Thus, the resulting preparation is highly safe.

### Brief Description of the Drawings

Fig.1 shows the result of fluorescence microscopic observation of Comparative Example 1.
Fig.2 shows the result of fluorescence microscopic observation of Comparative Example 2.
Fig.3 shows the result of fluorescence microscopic observation of Comparative Example 3.
Fig.4 shows the result of fluorescence microscopic observation of Experimental Example 2.
Fig.5 shows the result of fluorescence microscopic observation of Comparative Example 4.
Fig.6 shows the result of fluorescence microscopic observation of Experimental Example 3.

### Best Mode for Carrying Out the Invention

Hereafter, embodiments of the present invention will be specifically described.

The anti-acne skin agent for external use of the present invention is characterized in that it comprises protein nanoparticles containing an active ingredient.

Types of active ingredients used in the present invention are not particularly limited, as long as such ingredients are absorbed through the skin so as to exhibit acne-treating activity. Preferred examples of such an active ingredient can be selected from among antioxidant components, moisturizing components, reactive oxygen-removing agents, anti-inflammatory agents, antibacterial and/or bactericidal agents, sebum-suppressing and/or absorbing agents, anti-aging agents, collagen synthesis promoters, anti-wrinkle agents, vitamins, minerals, and amino acids.

Examples of antioxidants include carotenes, retinoic acid, retinol, vitamin C and derivatives thereof, kinetin, astaxanthin, tretinoin, vitamin E and derivatives thereof, sesamin, α-lipoic acid, coenzyme Q10, flavonoids, erythorbic acid, gallic acid propyl, BHT (di-n-butylhydroxytoluene), BHA (butylhydroxyanisole), *Engelhardtia chrysolepis Hance* extract, soybean extract, black tea extract, green tea extract, and *Rosae multiflorae fructus* extract.

Examples of moisturizing components include agar, diglycerin, distearyldimonium hectorite, butylene glycol, polyethylene glycol, propylene glycol, hexylene glycol, *Coix lachrma-jobi* extract, vaseline, urea, hyaluronic acid, ceramide, Lipidure, isoflavone, amino acid, collagen, mucopolysaccharide, fucoidan, lactoferrin, sorbitol, chitin/chitosan, malic acid, glucuronic acid, placenta extract, seaweed extract, moutan cortex extract, sweet tea extract, hypericum extract, coleus extract, *Euonymus japonicus* extract, safflower extract, Rosa rugosa flower extract, *Polyporus sclerotium* extract, hawthorn extract, rosemary extract, duke extract, chamomile extract, *Lamium album* extract, *Litchi Chinensis* extract, *Achillea millefolium* extract, aloe extract, marronnier extract, *Thujopsis dolabrata* extract, Fucus extract, Osmoin extract, oat bran extract, tuberosa polysaccharide, *Cordyceps sinensis* (plant worm) extract, barley extract, orange extract, *Rehmannia glutinosa* extract, zanthoxylumb extract, and *Coix lachrma-jobi* extract. In addition, in the cases of casein nanoparticles, casein itself has moisture retention capacity.

Examples of reactive oxygen-removing agents include superoxide dismutase (SOD), mannitol, carotenoids such as beta carotene, astaxanthin, rutin and the derivative thereof, bilirubin, cholesterol, tryptophan, histidine, quercetin, quercitrin, catechin, catechin derivatives, gallic acid, gallic acid derivatives, *Scutellariae radix* extract, ginkgo extract, *Saxifraga stolonifera* (strawberry geranium) extract, melissa extract, *Geranium thunbergii* extract, moutan cortex extract, parsley extract, tormentilla extract, *Momordica grosvenori* extract, seaweed extract, *"Yashajitsu" (Alnus firma Sieb. et Zucc.)* extract, and Lycii cortex extract.

Examples of anti-inflammatory agents include: compounds and derivatives and salts thereof selected from the group consisting of azulene, guaiazulene, diphenhydramine hydrochloride, hydrocortisone acetate, predonisolone, glycyrrhizic acid, glycyrrhetinic acid, mefenamic acid, phenylbutazone, indomethacin, ibuprofen, and ketoprofen; and plant extracts selected from the group consisting of *Scutellariae radix* extract, *Artemisia capillaris* extract, balloonflower *(Platycodon grandiflorus*) extract, *Armeniacae semen* extract, gardenia extract, *Sasa veitchii* extract, gentiana extract, comfrey extract, white birch extract, mallow extract, *Persicae semen* extract, peach leaf extract, and *Eriobotryae folium* extract.

Examples of antibacterial and/or bactericidal agents include piroctone olamine, isopropyl methyl ether, hinokitiol, zinc pyrithione, climbazole, benzalkonium chloride, photosensitive pigment No. 101, chlorhexidine, salicylic acid, phenol, ketoconazole, miconazole, sulfur, triclosan, trichlorocarbanilide, chlorhexidine hydrochloride, chlorhexidine gluconate, halocarban, chlorophenesin, benzethonium chloride, benzalkonium chloride, lysozyme chloride, alkyldiaminoethyl glycine hydrochloride, isopropylmethylphenol, benzoic acid, photosensitive pigment No. 201, thymol, hexachlorophene, berberine, thioxolone, *Saxifraga stolonifera* (strawberry geranium) extract, Phellodendron Bark extract, and *Scutellariae radix* extract, the derivative thereof and the salt thereof.

Examples of sebum-suppressing and/or absorbing agents include burdock extract, fat-soluble burdock extract, peony root extract, sage extract, Calendula officinalis, linden extract, cropweed extract, pyridoxine hydrochloride, pyridoxine dicaprylate, pyridoxine dipalmitate, pyridoxine glycyrrhizinate, pyridoxine dilaurate, pyridoxine tripalmitate, pyridoxal phosphate, HP-β-CD, mica, pearl agent, talc, iron oxide, and titanium oxide, the derivative thereof and the salt thereof.

Examples of anti-aging agents include retinoic acid, retinol, vitamin C and the derivative thereof, kinetin, β-carotene, astaxanthin, and tretinoin.

Also, known components can be used as collagen synthesis promoters, anti-wrinkle agents, vitamins, minerals, or amino acids.

The above active ingredients may be used alone or in combinations of two or more.

According to the present invention, it was found that, with the use of interaction between a fat-soluble active ingredient and a casein hydrophobic domain, it is possible for casein nanoparticles to contain the active ingredient. Further, it was found that such particles remain stable in an aqueous solution. The ClogP of such component is preferably more than 0, more preferably 1 or more, and further preferably 3 or more.

Further, it was found that a particle mixture of casein and ionic polysaccharide or another ionic protein can contain an ionic active ingredient.

The anti-acne skin agent for external use of the present invention comprises preferably 0.01% to 50% by weight and most preferably 0.1% to 10% by weight protein nanoparticles. The anti-acne skin agent for external use of the present invention contains an active ingredient in a weight that is preferably 0.1 % to 100% and more preferably 0.1% to 50% of the protein weight.

According to the present invention, an active ingredient may be added during or after protein nanoparticle formation.

The average particle size of protein nanoparticles used in the present invention is generally 1 to 100 nm, preferably 10 to 100 nm, more preferably 10 to 50 nm, further preferably 10 to 40 nm, and particularly preferably 20 to 40 nm.

The type of protein used in the present invention is not particularly limited. However, a protein having a lysine residue and a glutamine residue is preferable. In addition, such protein having a molecular weight of approximately 10,000 to 1,000,000 is preferably used. The origin of the protein is not particularly limited. However, a naturally occurring protein, particularly a human-derived protein, is preferably used. Specific examples of a protein that can be used include at least one selected from the group consisting of collagen, gelatin, acid-treated gelatin, albumin, ovalbumin, casein, transferrin, globulin, fibroin, fibrin, laminin, fibronectin, and vitronectin. However, the compound used in the present invention is not limited to the aforementioned compounds. In addition, the origin of the protein is not particularly limited. Thus, bovine, swine, fish or plant protein, as well as recombinant protein of any thereof, can be used. Examples of recombinant gelatin that can be used include, but are not limited to, gelatins described in EP1014176 A2 and US Patent No. 6,992,172. Among them, casein, acid-treated gelatin, collagen, or albumin is preferable. Further, casein or acid-treated gelatin is most preferable. When casein is used in the present invention, the origin of the casein is not particularly limited. Casein may be milk-derived or bean-derived. Any of α-casein, β-casein, γ-casein, and κ-casein, as well as a mixture thereof, can be used. Caseins may be used alone or in combinations of two or more.

Proteins used in the present invention may be used alone or in combinations of two or more.

According to the present invention, a protein can be subjected to crosslinking treatment during and/or after nanoparticle formation. For the crosslinking treatment, an enzyme can be used. Any enzyme may be used without particular limitation as long as it has been known to have an action of causing protein crosslinking. Among such enzymes, transglutaminase is preferable.

Transglutaminase may be derived from a mammal or a microorganism. A recombinant transglutaminase can be used. Specific examples thereof include the Activa series by Ajinomoto Co., Inc., commercially available mammalian-derived transglutaminase serving as a reagent, such as guinea pig liver-derived transglutaminase, goat-derived transglutaminase, rabbit-derived transglutaminase, or human-derived recombinant transglutaminase produced by, for example, Oriental Yeast Co., Ltd., Upstate USA Inc., and Biodesign International.

The amount of an enzyme used for the crosslinking treatment in the present invention can be adequately determined depending upon protein type. In general, an enzyme can be added in a weight that is 0.1% to 100% and preferably approximately 1% to 50% of the protein weight.

The duration for an enzymatic crosslinking reaction can be adequately determined depending upon protein type and nanoparticle size. However, in general, the reaction can be carried out for 1 to 72 hours, and preferably 2 to 24 hours.

The temperature for an enzymatic crosslinking reaction can be adequately determined depending upon protein type and nanoparticle size. In general, the reaction can be carried out at 0°C to 80°C and preferably at 25°C to 60°C.

Enzymes used in the present invention may be used alone or in combinations of two or more.

Nanoparticles of the present invention can be prepared in accordance with Patent Document: JP Patent Publication (Kokai) No. 6-79168 A (1994); or C. Coester, Journal Microcapsulation, 2000, vol. 17, pp. 187-193, provided that an enzyme is preferably used instead of glutaraldehyde for a crosslinking method.

In addition, according to the present invention, the enzymatic crosslinking treatment is preferably carried out in an organic solvent. The organic solvent used herein is preferably an aqueous organic solvent such as ethanol, isopropanol, acetone, or THF.

Further, according to the present invention, it is preferable to remove an organic solvent by distillation subsequent to a crosslinking treatment, followed by water dispersion. It is also possible to add water prior to or subsequent to removal of an organic solvent by distillation.

It is also possible to add at least one component selected from the group consisting of lipids (e.g., phospholipid), anionic polysaccharides, cationic polysaccharides, anionic proteins, cationic proteins, and cyclodextrin to the anti-acne skin agent for external use of the present invention. The amounts of lipid (e.g. phospholipid), anionic polysaccharide, cationic polysaccharide, anionic protein, cationic protein, and cyclodextrin to be added are not particularly limited. However, they can be added usually in a weight that is 0.1 % to 100% of the protein weight. In the case of the anti-acne skin agent for external use of the present invention, it is possible to adjust the release rate by changing the ratio of the above components to the protein.

Specific examples of phospholipids that can be used in the present invention include, but are not limited to, the following compounds: phosphatidylcholine (lecithin), phosphatidylethanolamine, phosphatidylserine, phosphatidylinositol, phosphatidylglycerol, diphosphatidylglycerol, and sphingomyelin.

Anionic polysaccharides that can be used in the present invention are polysaccharides having an acidic polar group such as a carboxyl group, a sulfate group, or a phosphate group. Specific examples thereof include, but are not limited to, the following compounds: chondroitin sulfate, dextran sulfate, carboxymethyl cellulose, carboxymethyl dextran, alginic acid, pectin, carrageenan, fucoidan, agaropectin, porphyran, karaya gum, gellan gum, xanthan gum, and hyaluronic acids.

Cationic polysaccharides that can be used in the present invention are polysaccharides having a basic polar group such as an amino group. Examples thereof include, but are not limited to, the following compounds : polysaccharides such as chitin or chitosan, which comprise, as a monosaccharide unit, glucosamine or galactosamine.

Anionic proteins that can be used in the present invention are proteins and lipoproteins having a more basic isoelectric point than the physiological pH. Specific examples thereof include, but are not limited to, the following compounds: polyglutamic acid, polyaspartic acid, lysozyme, cytochrome C, ribonuclease, trypsinogen, chymotrypsinogen, and α-chymotrypsin.

Cationic proteins that can be used in the present invention are proteins and lipoproteins having a more acidic isoelectric point than the physiological pH. Specific examples thereof include, but are not limited to, the following compounds: polylysine, polyarginine, histone, protamine, and ovalbumin.

According to the present invention, it is possible to use casein nanoparticles prepared by the following steps (a) to (c) of:
(a) mixing casein with a basic aqueous medium at pH of 8 to less than 11;
(b) adding at least one active ingredient to the solution obtained in step (a); and
(c) injecting the solution obtained in step (b) into an aqueous medium at a pH of 3.5 to 7.5.

Further, according to the present invention, it is possible to use casein nanoparticles prepared by the following steps (a) to (c) of:
(a) mixing casein with a basic aqueous medium at a pH of 8 to less than 11;
(b) adding at least one active ingredient to the solution obtained in step (a); and
(c) lowering the pH of the solution obtained in step (b) to a pH value which is distinct from the isoelectric point by 1 unit or more.

According to the present invention, it is possible to prepare casein nanoparticles of desired sizes. Also, with the use of interaction between a hydrophobic active ingredient and a casein hydrophobic domain, it is possible for casein nanoparticles to contain the active ingredient. In addition, it was found that such particles remain stable in an aqueous solution.

Further, it was found that a particle mixture of casein and ionic polysaccharide or another ionic protein contains an ionic active ingredient.

The method for preparing casein nanoparticles of the present invention involves a method wherein casein is mixed with a basic aqueous medium solution and the solution is injected into another acidic aqueous medium, and a method wherein casein is mixed with a basic aqueous medium solution and the pH of the solution is lowered during stirring, for example.

The method wherein casein is mixed with a basic aqueous medium solution and the solution is injected into another acidic aqueous medium is preferably carried out using a syringe for convenience. However, there is no particular limitation as long as the injection rate, solubility, temperature, and stirring conditions are satisfied. Injection can be carried out usually at an injection rate of 1 mL/min to 100 mL/min. The temperature of the basic aqueous medium can be adequately determined. In general, the temperature is 0°C to 80°C and preferably 25°C to 70°C. The temperature of an acidic aqueous medium can be adequately determined. In general, the temperature can be 0°C to 80°C and preferably 25°C to 60°C. The stirring rate can be adequately determined. However, in general, the stirring rate can be 100 rpm to 3000 rpm and preferably 200 rpm to 2000 rpm.

In the method wherein casein is mixed with a basic aqueous medium solution and the pH of the medium is lowered during stirring, it is preferable to add acid dropwise for convenience. However, there is no particular limitation as long as solubility, temperature, and stirring conditions are satisfied. The temperature of a basic aqueous medium can be adequately determined. However, in general, the temperature can be 0°C to 80°C and preferably 25°C to 70°C. The stirring rate can be adequately determined. However, in general, the stirring rate can be 100 rpm to 3000 rpm and preferably 200 rpm to 2000 rpm.

The aqueous medium that can be used for the present invention is an aqueous solution or a buffer comprising an organic acid or base or an inorganic acid or base.

Specific examples thereof include, but are not limited to, aqueous solutions comprising: organic acids such as citric acid, ascorbic acid, gluconic acid, carboxylic acid, tartaric acid, succinic acid, acetic acid, phthalic acid, trifluoroacetic acid, morpholinoethanesulfonic acid, and 2-[4-(2-hydroxyethyl)-1-piperazinyl]ethanesulfonic acid; organic bases such as tris (hydroxymethyl), aminomethane, and ammonia; inorganic acids such as hydrochloric acid, perchloric acid, and carbonic acid; and inorganic bases such as sodium phosphate, potassium phosphate, calcium hydroxide, sodium hydroxide, potassium hydroxide, and magnesium hydroxide.

The concentration of an aqueous medium used in the present invention is preferably approximately 10 mM to 1 M, and more preferably approximately 20 mM to 200 mM.

The pH of a basic aqueous medium used in the present invention is preferably 8 or more, more preferably 8 to 12, and further preferably 9 to 11. When the pH is excessively high, there is concern regarding hydrolysis or risks in handling. Thus, the pH is preferably in the above range.

According to the present invention, the temperature at which casein is mixed with a basic aqueous medium at pH of 8 or more is preferably 0°C to 80°C, more preferably 10°C to 60°C, and further preferably 20°C to 40°C.

The pH of an acidic aqueous medium used in the present invention is preferably 3.5 to 7.5 and more preferably 5 to 6.

The anti-acne skin agent for external use of the present invention may also comprise additives. The types of such additives are not particularly limited. One or more types selected from among a skin-lightening agent, an ultraviolet absorbing agent, a softening agent, a transdermal absorption enhancer, a soothing agent, a preservative, an antioxidant, a coloring agent, a thickener, an aroma chemical, and a pH adjuster, can be used.

Examples of skin-lightening agents used in the present invention include arbutin, L-ascorbic acid and the derivative thereof, hydroquinone, glutathione, the derivative thereof and the salt thereof, placenta extract, tranexamic acid and the derivative thereof glucoside, alkoxysalicylic acid and the salt thereof, Kojic acid and the derivative thereof, cysteine and the derivative thereof, ellagic acid, a resorcin derivative such as lucinol, chamomile extract, glycyrrhiza extract, *Inula britannica* extract, Prickly Restharrow extract, *Cassia mimosoides* extract, Mulberry bark extract, Japanese angelica root extract, Bistorta major extract, Sophora root extract, hawthorn extract, white lily extract, hop extract, Rosa polyantha extract, Rosa rugosa extract, *Acanthopanax spinosus* extract, *Chaenomelis fructus* extract, muscovado extract, wheat germ extract, *Artemisiae capillaris flos* extract, coix seed extract, *Aralia elata* extract, *Althaea officinalis* extract, and grape seed extract.

Specific examples of ultraviolet absorbing agents that can be used in the present invention include, but are not limited to, the following compounds according to the present invention: homomenthyl salicylate, 4-methoxycinnamic acid-2-ethylhexyl, 2-hydroxy-4-methoxy benzophenone, 2-hydroxy-4-methoxy benzophenone sulfonic acid, 2-hydroxy-4-methoxy benzophenone sodium sulfonate, 4-t-butyl-4'-methoxy-dibenzoylmethane, titanium oxide, and zinc oxide.

Specific examples of softening agents that can be used in the present invention include, but are not limited to, the following compounds according to the present invention: glycerin, mineral oil, and emollient ingredients (e.g., isopropyl isostearate, polyglyceryl isostearate, isotridecyl isononanoate, octyl isononanoate, oleic acid, glyceryl oleate, cocoa butter, cholesterol, mixed fatty acid triglyceride, dioctyl succinate, sucrose tetrastearate triacetate, cyclopentasiloxane, sucrose distearate, octyl palmitate, octyl hydroxystearate, arachidyl behenate, sucrose polybehenate, polymethylsilsesquioxane, myristyl alcohol, cetyl myristate, myristyl myristate, hexyl laurate, etc.).

Specific examples of transdermal absorption enhancers that can be used in the present invention include, but are not limited to, the following compounds according to the present invention: ethanol, isopropyl myristate, citric acid, squalane, oleic acid, menthol, N-methyl-2-pyrrolidone, diethyl adipate, diisopropyl adipate, diethyl sebacate, diisopropyl sebacate, isopropyl palmitate, isopropyl oleate, octyldodecyl oleate, isostearyl alcohol, 2-octyldodecanol, urea, vegetable oil, and animal oil.

Specific examples of soothing agents that can be used in the present invention include, but are not limited to, the following compounds according to the present invention: benzyl alcohol, procaine hydrochloride, xylocaine hydrochloride, and chlorobutanol.

Specific examples of preservatives that can be used in the present invention include, but are not limited to, the following compounds according to the present invention: benzoic acid, sodium benzoate, paraben, ethylparaben, methylparaben, propylparaben, butylparaben, potassium sorbate, sodium sorbate, sorbic acid, sodium dehydroacetate, hydrogen peroxide, formic acid, ethyl formate, sodium hypochlorite, propionic acid, sodium propionate, calcium propionate, pectin degradation products, polylysine, phenol, isopropylmethyl phenol, orthophenylphenol, phenoxyethanol, resorcin, thymol, thiram, and tea tree oil.

Specific examples of antioxidants that can be used in the present invention include, but are not limited to, the following compounds according to the present invention: vitamin A, retinoic acid, retinol, retinol acetate, retinol palmitate, retinyl acetate, retinyl palmitate, tocopheryl retinoate, vitamin C and the derivative thereof, kinetin, β-carotene, astaxanthin, lutein, lycopene, tretinoin, vitamin E, α-lipoic acid, coenzyme Q10, polyphenol, SOD, and phytic acid.

Specific examples of coloring agents that can be used in the present invention include, but are not limited to, the following compounds according to the present invention: krill pigment, orange dye, cacao dye, kaoline, carmines, ultramarine blue, cochineal dye, chrome oxide, iron oxide, titanium dioxide, tar dye, and chlorophyll.

Specific examples of thickeners that can be used in the present invention include, but are not limited to, the following compounds according to the present invention: quince seed, carrageenan, gum arabic, karaya gum, xanthan gum, gellan gum, tamarind gum, locust bean gum, gum traganth, pectin, starch, cyclodextrin, methylcellulose, ethylcellulose, carboxymethylcellulose, sodium alginate, polyvinyl alcohol, polyvinyl pyrrolidone, carboxyvinyl polymer, and sodium polyacrylate.

Specific examples of aroma chemicals that can be used in the present invention include, but are not limited to, the following compounds according to the present invention: musk, acacia oil, anise oil, ylang ylang oil, cinnamon oil, jasmine oil, sweet orange oil, spearmint oil, geranium oil, thyme oil, neroli oil, mentha oil, hinoki (Japanese cypress) oil, fennel oil, peppermint oil, bergamot oil, lime oil, lavender oil, lemon oil, lemongrass oil, rose oil, rosewood oil, anisaldehyde, geraniol, citral, civetone, muscone, limonene, and vanillin.

Specific examples of pH adjusters that can be used in the present invention include, but are not limited to, the following compounds according to the present invention: sodium citrate, sodium acetate, sodium hydroxide, potassium hydroxide, phosphoric acid, and succinic acid.

The dosage form of the anti-acne skin agent for external use of the present invention is not particularly limited. Examples of the dosage form include, but are not limited to, liquid formulations, fomentations, embrocations, gels, creams, aerosols, lotions, powders, foaming agents, skin lotions, body soap, soap, and cosmetics.

The anti-acne skin agent for external use of the present invention can be transdermally or transmucosally administered.

The dose of the anti-acne skin agent for external use of the present invention can be adequately determined depending on type and amount of active ingredient, user's body weight, and disease conditions, for example. The dose for single administration is generally approximately 1 µg to 50 mg/cm², and preferably 2.5 µg to 10 mg/cm².

The present invention is hereafter described in greater detail with reference to the following examples, although the technical scope of the present invention is not limited thereto.

### Examples

### Example 1:

Milk-derived casein (100 mg; Wako Pure Chemical Industries, Ltd.) was mixed with 50 mM phosphate buffer (pH 10, 10 mL). Coumarin 6 (0.015 mg; Wako Pure Chemical Industries, Ltd.) was dissolved in ethanol (0.1 mL). The two different solutions were mixed together. Hydrochloric acid was added thereto so that the pH was adjusted to 7.5. Thus, casein nanoparticles were obtained.

The average particle size of the above particles was measured with "Nanotrac," a light scattering photometer (NIKKISO Co., Ltd.), and it was found to be 29 nm.

### Example 2:

Milk-derived casein Na (10 mg; Wako Pure Chemical Industries, Ltd.) was mixed with a 50 mM phosphate buffer (pH 9; 1 mL). Glycyrrhetic acid (1.7 mg; Wako Pure Chemical Industries, Ltd.) was dissolved in ethanol (0.25 mL). The glycyrrhetic acid solution was added dropwise to the casein solution during stirring. The resulting liquid mixture (1 ml) was injected into 200 mM phosphate buffer water (pH 5; 10 mL) with the use of a microsyringe at an external temperature of 40°C during stirring at 800 rpm. Thus, a water dispersion of casein nanoparticles containing glycyrrhetic acid was obtained.

The average particle size of the above particles was measured with "Microtrac," a light scattering photometer (NIKKISO Co., Ltd.), and it was found to be 83 nm.

### Example 3:

Milk-derived casein (20 mg; Wako Pure Chemical Industries, Ltd.) and protamine sulfate (1 mg; Wako Pure Chemical Industries, Ltd.) were mixed with a 50 mM phosphate buffer (pH 10; 1 mL). α-lipoic acid (1 mg) was dissolved in ion-exchange water. The two types of solutions were mixed together. The resulting liquid mixture (1 ml) was injected into 200 mM phosphate buffer water (pH 5; 10 mL) with the use of a microsyringe at an external temperature of 40°C during stirring at 800 rpm. Thus, casein nanoparticles were obtained.

The average particle size of the above particles was measured with "Nanotrac," a light scattering photometer (NIKKISO Co., Ltd.), and it was found to be 48 nm.

### Example 4:

Milk-derived casein Na (10 mg; Wako Pure Chemical Industries, Ltd.) was mixed with a 50 mM phosphate buffer (pH 9, 1 mL). Tocopherol acetate (1.7 mg) was dissolved in ethanol (0.25 mL). The tocopherol acetate solution was added dropwise to the casein solution during stirring. The resulting liquid mixture (1 ml: casein solution) was injected into 200 mM phosphate buffer water (10 mL) with the use of a microsyringe at an external temperature of 40°C during stirring at 800 rpm. Thus, a water dispersion of casein nanoparticles containing tocopherol acetate was obtained.

The average particle size of the above particles was measured with "Microtrac," a light scattering photometer (NIKKISO Co., Ltd.), and it was found to be 124 nm.

### Example 5:

Ethanol (0.5 mL) and milk-derived casein (20 mg; Wako Pure Chemical Industries, Ltd.) were mixed with a 50 mM phosphate buffer (pH 10; 1 mL). β-carotene (0.5 mg; Wako Pure Chemical Industries, Ltd.) was dissolved in ethanol (0.25 mL). The β-carotene solution was added dropwise to the casein solution during stirring. The resulting liquid mixture was injected into 300 mM phosphate buffer water (pH 5; 10 mL) with the use of a microsyringe at an external temperature of 40°C during stirring at 800 rpm. Thus, casein nanoparticles were obtained.

The average particle size of the above particles was measured with "Nanotrac," a light scattering photometer (NIKKISO Co., Ltd.), and it was found to be 88 nm.

### Example 6:

Acid-treated gelatin (10 mg) and TG-S (Transglutaminase, 5 mg; Ajinomoto Co., Inc.) were dissolved in water (1 mL). The gelatin solution (1 ml) was injected into ethanol (10 mL) in which glycyrrhetic acid (1.7 mg) had been dissolved, with the use of a microsyringe at an external temperature of 40°C during stirring at 800 rpm. Thus, gelatin nanoparticles were obtained. The gelatin nanoparticles were allowed to stand at an external temperature of 55°C for 5 hours for enzymatic crosslinking.

The average particle size of the above particles was measured with "Microtrac," a light scattering photometer (NIKKISO Co., Ltd.), and it was found to be 80 nm.

### Example 7:

Milk-derived casein Na (10 mg; Wako Pure Chemical Industries, Ltd.) was mixed with a phosphate buffer (pH 9; 1 mL). Palmitoylascorbic acid (1 mg; Wako Pure Chemical Industries, Ltd.) was dissolved in ethanol (0.2 mL). The two above solutions were mixed together. The resulting liquid mixture (1 ml; casein solution) was injected into phosphate buffer water (pH 5; 10 mL) with the use of a microsyringe at an external temperature of 40°C during stirring at 800 rpm. Thus, a water dispersion of casein nanoparticles containing palmitoylascorbic acid was obtained.

The average particle size of the above particles was measured with "Microtrac," a light scattering photometer (NIKKISO Co., Ltd.), and it was found to be 124 nm.

### Example 8:

Milk-derived casein (10 mg; Wako Pure Chemical Industries, Ltd.) was mixed with a 50 mM phosphate buffer (pH 10; 1 mL). Retinoic acid (1 mg) was dissolved in ethanol (0.3 mL). A 1% polylysine aqueous solution (0.2 mL) was added to the casein solution, and a retinoic acid solution was then added dropwise thereto during stirring. The resulting liquid mixture (1 ml; casein solution) was injected into 200 mM phosphate buffer water (pH 5; 10 mL) with the use of a microsyringe at an external temperature of 40°C during stirring at 800 rpm. Thus, a water dispersion of casein nanoparticles containing retinoic acid was obtained.

The average particle size of the above particles was measured with a light scattering photometer (DLS-7000; Otsuka Electronics Co., Ltd.), and it was found to be 69 nm.

### Example 9:

Acid-treated gelatin (10 mg) and TG-S (Transglutaminase, 5 mg; Ajinomoto Co., Inc.) were dissolved in water (1mL). The gelatin solution (1 ml) was injected into ethanol (10 mL) in which tocopherol (1.7 mg) had been dissolved, with the use of a microsyringe at an external temperature of 40°C during stirring at 800 rpm. Thus, gelatin nanoparticles were obtained. The gelatin nanoparticles were allowed to stand at an external temperature of 55°C for 5 hours for enzymatic crosslinking.

The average particle size of the above particles was measured with "Microtrac" a light scattering photometer (NIKKISO Co., Ltd.), and it was found to be 95 nm.

### Example 10:

Milk-derived casein (100 mg; Wako Pure Chemical Industries, Ltd.) was mixed with a 50 mM phosphate buffer (pH 10; 10 mL). Hinokitiol (7 mg) was dissolved in ethanol (0.1 mL). The two types of solutions were mixed, and hydrochloric acid was then added to the mixed solution, so that the pH of the solution was adjusted to pH 6, thereby obtaining casein nanoparticles.

The average particle size of the above particles was measured with a light scattering photometer (Nano-ZS; Malvern Instruments Ltd.), and it was found to be 23 nm.

### Experimental example 1:

The dispersions of nanoparticles containing hair-growth drugs described in Examples 2 to 6 were preserved at room temperature for 1 month. Thereafter, the average particle size was measured using a Microtrac (NIKKISO Co., Ltd.).

As Comparative example 1, "NanoImpact" (Hosokawa Micron Corporation), a synthetic polymer (PLGA) nanoparticle dispersion, was used.

Table 1 shows measurement results obtained in Experimental example 1.

**Table 1:**

| | Comparative example 1 | Example 2 | Example 3 | Example 4 | Example 5 | Example 6 |
|---|---|---|---|---|---|---|
| When prepared | 600 nm | 83 nm | 48 nm | 124 nm | 88 nm | 80 nm |
| 1 month later | N. D. | 90 nm | 63 nm | 141 nm | 90 nm | 93 nm |

| | | | | | | |
|---|---|---|---|---|---|---|
| N. D.: Not detected | | | | | | |

### Experimental example 2: Hairless rat excised skin test

A 2-cm square piece of nonwoven cloth absorbing 400 µl of the casein nanoparticle dispersion prepared in Example 1 was applied to hairless rat excised skin, and the skin was then allowed to stand for 30 minutes. Thereafter, the skin was embedded within an OCT compound (Sakura Finetek Co., Ltd.), and it was then frozen with liquid nitrogen. A frozen section was prepared from the aforementioned skin with the use of a cryostat (Carl Zeiss). The section was immobilized and enclosed on a prepared slide with the use of a DAPI-containing mounting agent, followed by fluorescence microscopic observation.

### Comparative example 1: No application

### Comparative example 2:

With the use of the following dispersion, fluorescence microscopic observation was conducted in the same manner as in Experimental example 2.

Milk-derived casein (100 mg; Wako Pure Chemical Industries, Ltd.) was mixed with distilled water (10 mL). Coumarin 6 (0.015 mg; Wako Pure Chemical Industries, Ltd.) was dissolved in ethanol (0.1 mL). A dispersion was obtained by mixing the two types of solutions.

### Comparative example 3:

With the use of the following solution, fluorescence microscopic observation was conducted in the same manner as in Experimental example 2.

A solution was obtained by dissolving coumarin 6 at a concentration of 0.15 mg/mL in a 50% ethanol aqueous solution.

### Experimental example 3: SD rat in vivo test

A SD rat was subjected to anesthetic injection. Thereafter, a 2-cm square piece of nonwoven cloth absorbing 400 µl of the casein nanoparticle dispersion prepared in Example 1 was applied to the abdominal skin of the rat, and the skin was then allowed to stand for 60 minutes. The skin was embedded within an OCT compound (Sakura Finetek Co., Ltd.), and it was then frozen with liquid nitrogen. A frozen section was prepared from the aforementioned skin with the use of a cryostat (Carl Zeiss). Thereafter, the section was immobilized and enclosed on a prepared slide with the use of a DAPI-containing mounting agent, followed by fluorescence microscopic observation.

### Comparative example 4:

With the use of the following solution, fluorescence microscopic observation was conducted in the same manner as in Experimental example 3.

A solution was obtained by dissolving coumarin 6 at a concentration of 0.15 mg/mL in a 50% ethanol aqueous solution.

Figs. 1a to 6a show fluorescence photomicrographs of the hairless rat excised skins and the SD rat skin sections used in Comparative examples 1, 2, 3 and 4, and Experimental examples 2 and 3. Figs. 1b to 6b show DAPI-stained tissue images corresponding to the visual fields of Figs. 1a to 6a.

Based on the above Examples, it is understood that the anti-acne skin agent for external use of the present invention has a high permeability to skin and pore and a high safety. Further, it contains natural polymers so as to be highly safe. Furthermore, it has a small particle diameter so as to have high transparency.

Still further, the anti-acne skin agent for external use of the present invention was evaluated by 5 trial volunteers. They all evaluated that the aforementioned anti-acne skin agent was excellent for use in terms of ease of application, fresh sensation on the skin, no stickiness, moisturizing feeling, and the like.

## Claims

1. An anti-acne skin agent for external use, which comprises protein nanoparticles containing an active ingredient.

2. The anti-acne skin agent for external use according to claim 1, wherein the average particle size of the protein nanoparticles is 10 to 100 nm.

3. The anti-acne skin agent for external use according to claim 1 wherein the protein nanoparticles contain an active ingredient in a weight that is 0.1 % to 100% of the protein weight.

4. The anti-acne skin agent for external use according to claim 1 wherein the active ingredient is at least one selected from the group consisting of antioxidant components, moisturizing components, reactive oxygen-removing agents, anti-inflammatory agents, antibacterial and/or bactericidal agents, sebum-suppressing and/or absorbing agents, anti-aging agents, collagen synthesis promoters, anti-wrinkle agents, vitamins, minerals, and amino acids.

5. The anti-acne skin agent for external use according to claim 1 wherein the active ingredient is an ionic substance or a fat-soluble substance.

6. The anti-acne skin agent for external use according to claim 1 wherein the protein is at least one selected from the group consisting of collagen, gelatin, acid-treated gelatin, albumin, ovalbumin, casein, transferrin, globulin, fibroin, fibrin, laminin, fibronectin, and vitronectin.

7. The anti-acne skin agent for external use according to claim 1 wherein the protein is subjected to crosslinking treatment during and/or after nanoparticle formation.

8. The anti-acne skin agent for external use according to claim 7 wherein crosslinking treatment is carried out by an enzyme.

9. The anti-acne skin agent for external use according to claim 1 which comprises casein nanoparticles prepared by the following steps (a) to (c):
(a) mixing casein with a basic aqueous medium at pH of from 8 to less than 11;
(b) adding at least one active ingredient to the solution obtained in step (a); and
(c) injecting the solution obtained in step (b) into an aqueous medium at pH of 3.5 to 7.5:

10. The anti-acne skin agent for external use according to claim 1 which comprises casein nanoparticles prepared by the following steps (a) to (c):
(a) mixing casein with a basic aqueous medium at pH of from 8 to less than 11;
(b) adding at least one active ingredient to the solution obtained in step (a); and
(c) lowering the pH of the solution obtained in step (b) to pH value which is different from the isoelectric point by 1 or more units.
